# EUROPEAN PATENT SPECIFICATION

(11) **EP 2 478 928 B1**
(45) Date of publication and mention of the grant of the patent: **24.07.2019**
(21) Application number: 10817052.3
(22) Date of filing: 02.09.2010
(51) Int. Cl.: A61M 25/00, A61M 25/01

(54) **CATHETER HOLDER**
KATHETERHALTER
PORTE-CATHÉTER

(30) Priority: 16.09.2009 JP 2009214998
(43) Date of publication of application: 25.07.2012
(73) Proprietor: Terumo Kabushiki Kaisha, Tokyo 151-0072 (JP)
(72) Inventor: NAKAMOTO, Ryou, Ashigarakami-gun Kanaga-wa, 259-0151 (JP); MITSUHASHI, Kenta, Fujinomiya-shi Shizuoka 418-0015 (JP)
(74) Representative: Dossmann, Gérard
(86) International application number: PCT/JP2010/065021
(87) International publication number: WO 2011/033939

(56) References cited:
- WO-A1-99/47202
- JP-A- 10 286 263
- JP-A- H11 332 992
- JP-A- 2001 505 449
- JP-A- 2004 290 395
- JP-A- 2004 524 912
- JP-A- 2008 510 579
- US-A1- 2007 276 333

## Description

### TECHNICAL FIELD

The present disclosure relates to a catheter holder used for packaging a catheter.

### BACKGROUND ART

As described, for example, in Patent Document 1, a catheter is generally housed in a holder tube wound roundly and then, is packaged in a state in which there is held an unhoused portion such as a connector, a portion of a sheath and the like which is exposed from the holder tube.

For a mainstream way of such a package, there exist a method of storing the whole catheter together with the holder tube in a bag by fixing them on a mat board, a method of setting the whole catheter together with the holder tube on a tray and a method of storing the catheter in a bag by fastening one place of the unhoused portion onto the holder tube.

Further catheter holders are known from the prior-art document WO 99/47202 A1.

### Prior-art Document

### Patent Document

Patent Document 1: Japanese unexamined patent publication No. 2004-290395

### DISCLOSURE OF THE INVENTION

However, in a method of using a mat board or a tray, since the whole catheter is received by a mat board or a tray, it is necessary to provide a mat board or a tray in conformity with the size of the whole catheter, so that the capacity of the member used for the package is large and is liable to be bulky as compared with that in the method of fastening one place of the unhoused portion onto the holder tube. On the other hand, in a method of fastening the unhoused portion onto the holder tube, the unhoused portion is held only by one place, so that it is difficult to hold the unhoused portion stably as compared with a case of using a mat board or a tray. In particular, for a highly functionalized catheter, the proximal portion thereof is heavy due to a factor such as a structure including an electrical connection portion or the like and also, there exists such a situation that the catheter is easily broken due to a factor such as a structure in which a signal path of an optical fiber or the like passes therethrough and the like, and it is difficult to package such a highly functionalized catheter by fastening one place of the unhoused portion onto the holder tube.

The present invention was invented so as to solve those problems and is addressed to provide a catheter holder which is compact and in which the unhoused portion of the catheter can be held stably.

A catheter holder of the present invention for achieving the object mentioned above includes: an attachment portion for being attached to a portion of a holder tube for housing a catheter; and a plurality of hold portions constituted integrally with the attachment portion and holding an unhoused portion of the catheter, which is exposed from the holder tube.

The catheter holder of the present invention constituted as described above includes an attachment portion to be attached on a portion of the holder tube, so that it is not necessary to employ a structure of being attached to the whole catheter as in a case of a mat board or a tray and it is compact and also, since a plurality of hold portions are included, it is possible to hold the unhoused portion of the catheter stably.

The plurality of hold portions are constituted so as to include a plurality of sandwiching portions for sandwiching the unhoused portion when closing a first member and a second member which are freely rotatable in a hinge form, the holding depending on the plurality of sandwiching portions is released at one time by opening the first member and the second member and the removal of the catheter is easy.

The catheter holder includes stopper portions provided at the first member and the second member wherein the stopper portions are engaged with each other for restriction so as not to open the first member and the second member, and they depart from each other for setting a state in which the first member and the second member become openable and closable, it is possible to prevent the first member and the second member from being opened unintentionally.

If the catheter holder is constituted so as to include engagement portions provided at the first member and the second member, which are engaged with each other by a mechanism in which the first member and the second member are closed, and which depart from each other by a mechanism in which the first member and the second member are opened, it is possible, by adjusting the engagement force of the engagement portions, to adjust the holding force for holding the unhoused portion and to adjust easiness of opening between the first member and the second member.

If the plurality of hold portions is constituted so as to include a plurality of concave portions to be fitted with the unhoused portion in which the cross-sectional shape thereof intersecting in the axial direction of the catheter forms an undercut shape, the holding depending on the plurality of concave portions is released at one time by bending the catheter holder and by broadening the plurality of concave portions, and the removal of the catheter is easy.

### BRIEF DESCRIPTION OF DRAWINGS

FIG. 1 is a perspective view showing a catheter holder, an unhoused portion and a holder tube of a first exemplified embodiment;
FIG. 2 is a perspective view showing the catheter holder of the first exemplified embodiment;
FIG. 3A is a partially enlarged perspective view showing a hold portion of the first exemplified embodiment by being opened;
FIG. 3B is a partially enlarged perspective view showing a state of housing the unhoused portion by closing the hold portion of the first exemplified embodiment;
FIG. 4A is a partially enlarged perspective view showing a stopper portion of the first exemplified embodiment by being enlarged;
FIG. 4B is a partially enlarged cross-sectional view showing the stopper portion of the first exemplified embodiment by being enlarged;
FIG. 5 is a partially enlarged perspective view showing an attachment portion of the first exemplified embodiment by being enlarged;
FIG. 6A is a perspective view showing a catheter holder of a second exemplified embodiment;
FIG. 6B is a partially enlarged cross-sectional view showing an engagement portion of the second exemplified embodiment by being enlarged;
FIG. 7A is a perspective view showing a state of housing an unhoused portion of a catheter holder of a third exemplified embodiment but not being part of the invention; and
FIG. 7B is a partially enlarged perspective view showing a hold portion of the third exemplified embodiment by being enlarged.

### BEST MODE FOR CARRYING OUT THE INVENTION

Hereinafter, exemplified embodiments of the present invention will be explained with reference to the drawings. It should be noted in the exemplified embodiments explained hereinafter that similar reference numerals are applied with respect to the members having functions common in the respective exemplified embodiments and in addition, repeated explanations thereof will be omitted.

### <First Exemplified Embodiment>

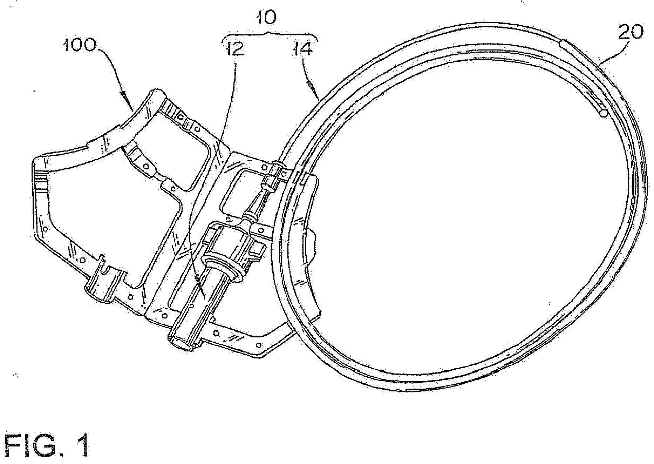

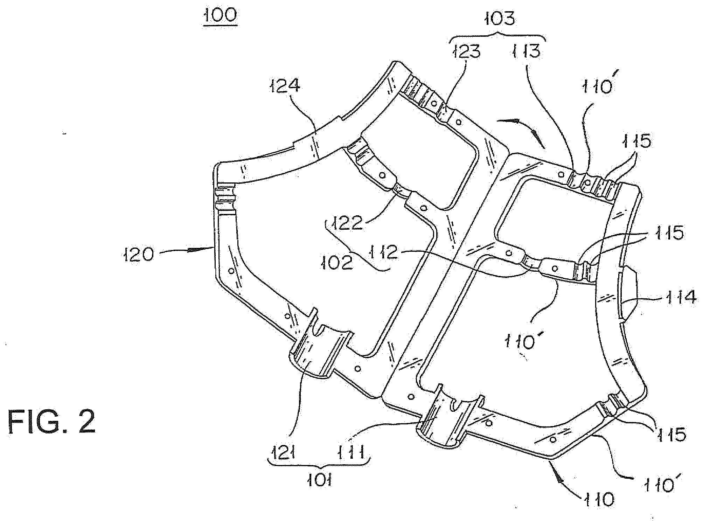

When the explanation is given in FIG. 1 and FIG. 2, a catheter holder 100 relating to a first exemplified embodiment is a holder used for packaging a catheter and includes a constituent piece 110 (first member) and a constituent piece 120 (second member) which are freely rotatable in a hinge form. The catheter holder 100 also includes claws 114, 124 (stopper portions) which are provided at the constituent piece 110 and the constituent piece 120 and which restrict those pieces so as not to be opened. Also, the catheter holder 100 includes concave portions 115 (attachment portions) which are attached to a portion of a holder tube 20 for housing the catheter and a plurality of hold portions 101, 102, 103 which are constituted integrally with the concave portions 115 and hold an unhoused portion 10 of the catheter, which is exposed from the holder tube 20 and not housed in the holder tube 20. The unhoused portion 10 includes a connector 12 for connecting the catheter with other apparatus, and a sheath 14 extending from the connector 12.

With respect to the three hold portions 101, 102, 103 of the catheter holder 100, one hold portion 101 holds the connector 12 and the other two hold portions 102, 103 hold the sheath 14. The hold portions 102, 103 hold the proximal portion of the sheath 14.

The plurality of hold portions 101, 102, 103 are a plurality of sandwiching portions for sandwiching the unhoused portion 10 by a mechanism in which the constituent piece 110 and the constituent piece 120 close. Then, the plurality of hold portions 101, 102, 103 are constituted by concave portions 111, 112, 113 provided at the constituent piece 110 and concave portions 121, 122, 123 provided at the constituent piece 120.

The concave portion 111 and the concave portion 121 have shapes matching with the region of the connector 12 which those concave portions hold. Similarly, the concave portion 113 and the concave portion 123 and also, the concave portion 112 and concave portion 122 have shapes matching with the regions of the sheath 14, which the respective concave portions will hold.

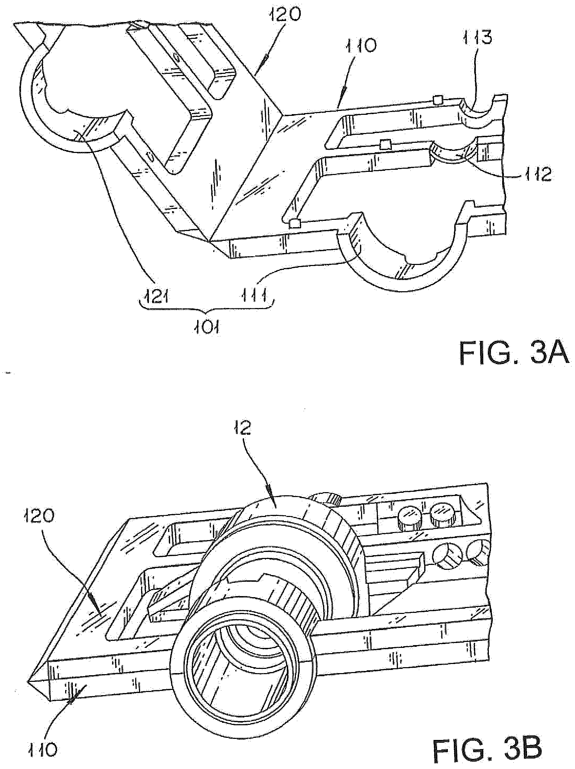

When the explanation is given in FIGS. 3 by taking the concave portion 111 and the concave portion 121 as an example, the region of the connector 12 which the concave portion 111 and the concave portion 121 hold is in a cylindrical shape, and by being combined together, the concave portion 111 and the concave portion 121 form a cylindrical hole portion with which the connector 12 is fitted. In addition, although not shown in the drawing, the configurations are similar for the concave portion 113 and the concave portion 123 and also, for the concave portion 112 and the concave portion 122, and those portions form hole portions with which the sheath 14 is to be fitted.

In each of the concave portion 111 and the concave portion 121, the cross-sectional shape thereof intersecting in the axial direction of the catheter is a semicircular shape. In addition, it is similar with respect to the concave portion 112 and the concave portion 122 and also, with respect to the concave portion 113 and the concave portion 123, the cross-sectional shapes thereof intersecting in the axial direction of the catheter are semicircular shapes. In other words, for the concave portions 111, 112, 113, 121, 122, 123, the cross-sectional shapes thereof intersecting in the axial direction of the catheter are not undercut shapes which surround the circumference of the catheter axis by an angle larger than 180° and less than 360°, and then, by opening the constituent piece 110 and the constituent piece 120, the hold portions 101, 102, 103 will lose the holding forces thereof.

With respect to the connector 12 or the sheath 14, the shape thereof differs depending on the region thereof, and there is included a level difference along with the axial direction of the catheter. Then, such a level difference is hooked on the catheter holder 100, specifically, on the concave portion 111, the concave portion 112 or the concave portion 113, so that even if the constituent piece 110 and the constituent piece 120 are opened and the holding force is lost, axial-direction deviation of the catheter, and further, dropping thereof are prevented. In order to prevent such deviation of the catheter, it is allowed to provide a protrusion on the outer circumferential surface of the connector 12 or of the sheath 14.

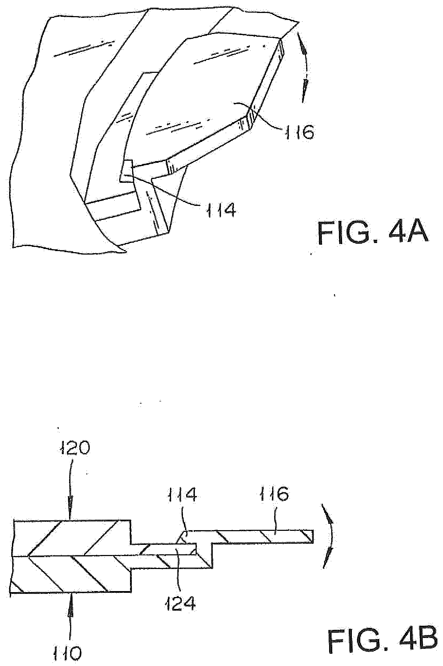

As shown in FIGS. 4, the claws 114, 124 are engaged with each other for restriction so as not to open the constituent piece 110 and the constituent piece 120, and they depart from each other for setting a state in which the constituent piece 110 and constituent piece 120 are openable and closable. The claw 114 of the one side is constituted integrally with a push portion 116 for detaching the claw 114. Then, by pressing the push portion 116, the claw 114 inclines and the hooking is released.

The catheter holder 100 includes one set of the claws 114, 124. It is allowed to include plural sets of the claws 114, 124, but merely unlatching a single set of the claws 114, 124 makes it possible to obtain a state in which the constituent piece 110 and the constituent piece 120 are made openable and closable, so that the operation thereof is simple as compared with a case of unlatching plural sets thereof and the removal of the catheter is easy.

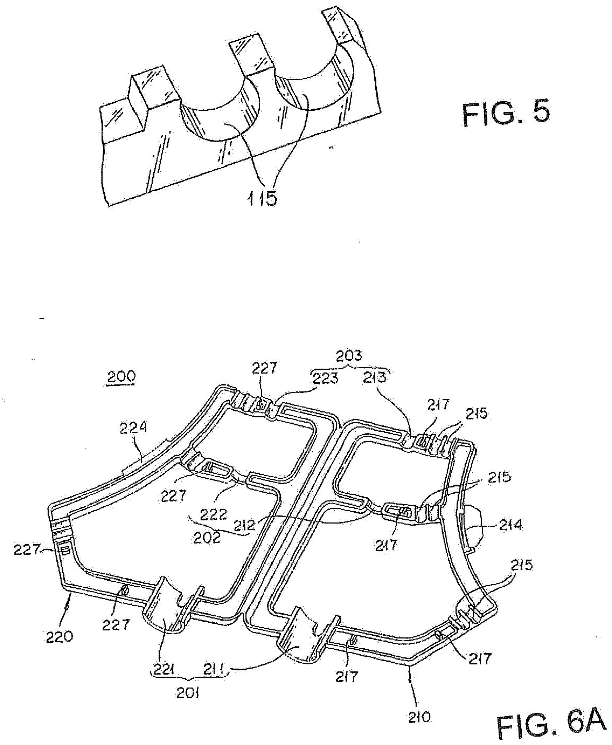

In the concave portion 115 such as shown in FIG. 5, the cross-sectional shape intersecting in the axial direction of the catheter does not form a semicircular shape but forms an undercut shape. The holder tube 20 and concave portion 115 are fitted and fixed to each other. Consequently, when opening the constituent piece 110 and the constituent piece 120, the holder tube 20 does not detach from the catheter holder 100 and remains in the catheter holder 100. It is possible to fit the holder tube 20 with the concave portion 115 by pushing the holder tube 20 into the concave portion 115 or by widening the concave portion 115 by bending the catheter holder 100.

Effects of the first exemplified embodiment will be described.

The catheter holder 100 includes the concave portion 115 to be attached on a portion of the holder tube 20, so that it is not necessary to employ a structure of being attached to the whole catheter as in a case of a mat board or a tray and it is compact and also, since the plurality of hold portions 101, 102, 103 are included, it is possible to hold the unhoused portion 10 of the catheter stably.

The plurality of hold portions 101, 102, 103 are a plurality of sandwiching portions for sandwiching the unhoused portion 10 when closing the constituent piece 110 and the constituent piece 120, so that the holding depending on the plurality of hold portions 101, 102, 103 is released at one time by opening the constituent piece 110 and the constituent piece 120 and the removal of the catheter is easy.

Also, in case of releasing the fitting between the constituent piece 110 and the constituent piece 120, as described above, it is constituted such that the holder tube 20 is not fitted on the concave portion 123 side but always on the concave portion 115 side. More specifically, in case of carrying out an action of releasing the fitting between the constituent pieces, the surgery operator can predict beforehand with which one of the constituent pieces the holder tube 20 is fitted, so that it is possible to carry out the removal of the catheter from the catheter holder efficiently.

Differently from this exemplified embodiment, in a case in which the plurality of hold portions are mutually independent and the releasings thereof are performed individually, it is necessary to hold the connector 12 and/or the sheath 14 every time when releasing each holding and the load thereof is exerted on the catheter repeatedly. On the other hand, in the catheter holder 100 of this exemplified embodiment, the plural holdings are released at one time and the load is not exerted on the catheter repeatedly, and it is possible to suppress the load on the catheter.

The claws 114, 124 are engaged with each other and restrict the constituent piece 110 and the constituent piece 120 so as not to be opened, and the claws depart from each other and establish a state in which the constituent piece 110 and the constituent piece 120 are openable and closable, so that it is possible to prevent the constituent piece 110 and the constituent piece 120 from being opened unintentionally.

### <Second Exemplified Embodiment>

The second exemplified embodiment is approximately similar to the first exemplified embodiment, but the second exemplified embodiment is different from the first exemplified embodiment in that there are included hooks 217, 227 (engagement portion) for adjusting the holding force for holding the unhoused portion 10.

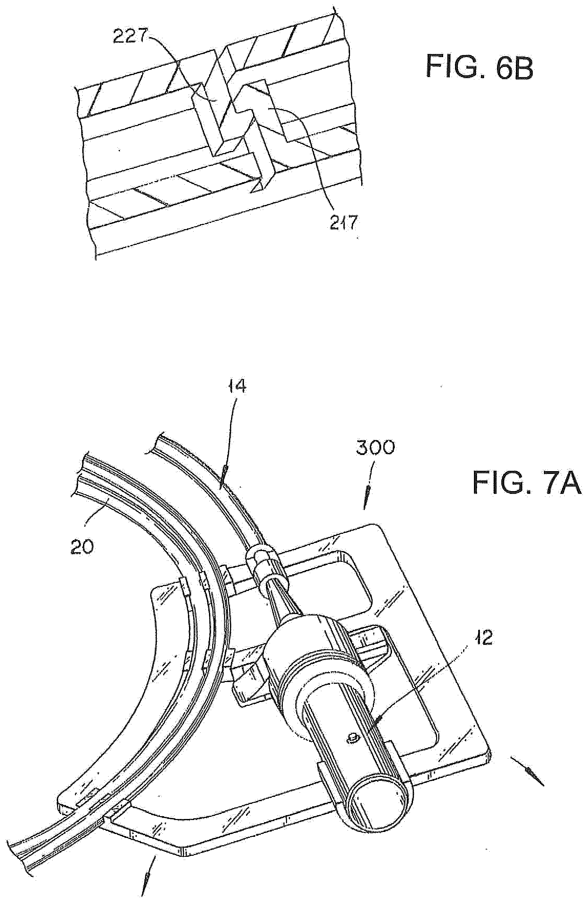

As shown in FIGS. 6, the hooks 217, 227 are provided at the constituent piece 210 and the constituent piece 220 and are engaged with each other by closing the constituent piece 210 and the constituent piece 220, and the hooks depart from each other by opening the constituent piece 210 and the constituent piece 220.

Since there are included such hooks 217, 227, it is possible for the catheter holder 200 of the second exemplified embodiment to adjust the holding force for holding the unhoused portion 10 and the easiness of opening between the constituent piece 210 and the constituent piece 220 by adjusting the engagement force between the hooks 217, 227. For example, the holding force can be intensified by intensifying the engagement force between the hook 217 and the hook 227 and conversely, it becomes easy to open the constituent piece 210 and the constituent piece 220 by weakening the engagement force between the hook 217 and the hook 227. In other words, the contradictory factors referred to as the holding force and the easiness of opening can be adjusted by changing the engagement force between the hook 217 and the hook 227.

### <Third Exemplified Embodiment but which is not part of the invention>

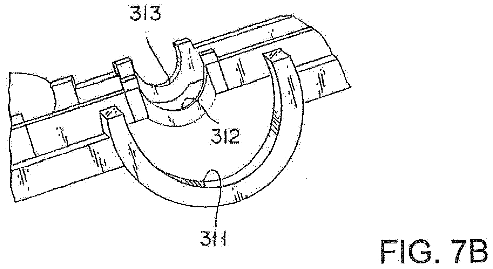

When the explanation is given in FIGS. 7, a catheter holder 300 of the third exemplified embodiment not being part of the invention includes a constituent piece approximately similar to the constituent piece 110 of the first exemplified embodiment but does not include the constituent piece 120, and the shapes of a plurality of concave portions 311, 312, 313 are different from those of the concave portions 111, 112, 113 of the constituent piece 110.

In each of the concave portions 311, 312, 313, the cross-sectional shape thereof intersecting in the axial direction of the catheter does not form a semicircular shape but forms an undercut shape. Then, the concave portions 311, 312, 313 are fitted with the connector 12 and the sheath 14, and hold them.

The catheter holder 300 includes such concave portions 311, 312, 313, so that by widening the plurality of concave portions 311, 312, 313 by bending the catheter holder 300, the holdings depending on the plurality of concave portions 311, 312, 313 are released at one time, and the removal of the catheter is easy. Also, it is possible to adjust the holding force for holding the unhoused portion 10 depending on the depth of the undercut.

The present invention is not limited by the exemplified embodiments described above and it is possible to apply modifications variously within the scope of the claims. For example, the places for holding the unhoused portion are not limited by three places and it is allowed to employ two places or employ places more then three. Also, the present invention covers a configuration in which a plurality of hold portions hold only the connector and a configuration in which a plurality of hold portions hold only the sheath.

Also, the present invention covers a configuration in which the concave portion of the third exemplified embodiment, which is to be fitted with the unhoused portion, is applied to the first and second exemplified embodiments. In other words, it is allowed for the hold portion to have a constitution including the sandwiching portion and the undercut-shaped concave portion. In this case, even if the first member and the second member are opened, the concave portion holds the unhoused portion, so that when opening the first member and the second member, it is possible to prevent the catheter from detaching from the catheter holder unintentionally. Also, when the undercut-shaped concave portion for holding the unhoused portion and the attachment portion are provided on the same one side within the first member and the second member, the catheter is not twisted when opening the first member and the second member, which is favorable.

In addition, the engagement portion is not limited by the hook and, for example, it is also allowed to employ a concave portion and a convex portion which are fitted with each other.

The subject-matter of the invention is defined by the appended claims.

### DESCRIPTION OF REFERENCE NUMERALS

10: unhoused portion
12: connector
14: sheath
20: holder tube
100, 200, 300: catheter holder
101, 102, 103: hold portion
110, 120, 210, 220: constituent piece (first member, second member)
115: concave portion (attachment portion)
114, 124: claw (stopper portion)
217, 227: hook (engagement portion)
311, 312, 313: concave portion

## Claims

1. A catheter holder (100, 200, 300) comprising:
an attachment portion (115) configured for being attached to a portion of a holder tube (20) for housing a catheter; and
further comprising
a plurality of hold portions (101, 102, 103) constituted integrally with the attachment portion (115) and configured for holding an unhoused portion (10) of the catheter, which is exposed from the holder tube (20),
wherein the plurality of hold portions (101, 102, 103) include a plurality of sandwiching portions configured for sandwiching the unhoused portion (10) when closing a first member (110, 210) and a second member (120, 220) which are freely rotatable in a hinge form,
**characterized by**
said catheter holder (100, 200, 300) comprising stopper portions (114, 124) provided at the first member (110, 210) and the second member (120, 220), wherein the stopper portions (114, 124) are configured to engage with each other for restriction so as not to open the first member (110, 210) and the second member (120, 220), and they are configured to depart from each other for setting a state in which the first member (110, 210) and the second member (120, 220) become openable and closable.

2. The catheter holder (100, 200, 300) according to claim 1, comprising engagement portions (217, 227) provided at the first member (110, 210) and the second member (120, 220), which are configured to engage with each other by a mechanism in which the first member (110, 210) and the second member (120, 220) are closed, and which are configured to depart from each other by a mechanism in which the first member (110, 210) and the second member (120, 220) are opened.

3. The catheter holder (100, 200, 300) according to claim 1 or claim 2, wherein the plurality of hold portions (101, 102, 103) include a plurality of concave portions (111, 112, 113, 121, 122, 123, 311, 312, 313) configured to be fitted with the unhoused portion (10) in which the cross-sectional shape thereof intersecting in the axial direction of the catheter forms an undercut shape.

## Patentansprüche

1. Katheterhalter (100, 200, 300), der Folgendes umfasst:
einen Befestigungsabschnitt (115), der dazu ausgebildet ist, an einem Abschnitt eines Halterohrs (20) zur Aufnahme eines Katheters befestigt zu werden; und
ferner Folgendes umfasst:
eine Vielzahl von Halteabschnitten (101, 102, 103), die einstückig mit dem Befestigungsabschnitt (115) ausgebildet sind und dazu dienen, einen ungehäusten Abschnitt (10) des Katheters zu halten, der gegenüber dem Halterohr (20) freiliegt,
wobei die Vielzahl von Halteabschnitten (101, 102, 103) eine Vielzahl von Einklemmabschnitten aufweisen, die dazu ausgebildet sind, beim Schließen eines ersten Elements (110, 210) und eines zweiten Elements (120, 220), die in Form eines Scharniers frei drehbar sind, den ungehäusten Abschnitt (10) zwischen sich aufzunehmen,
**dadurch gekennzeichnet, dass**
der Katheterhalter (100, 200, 300) Anschlagabschnitte (114, 124) aufweist, die an dem ersten Element (110, 210) und dem zweiten Element (120, 220) vorgesehen sind, wobei die Anschlagabschnitte (114, 124) dazu ausgebildet sind, zur Begrenzung ineinanderzugreifen, damit das erste Element (110, 210) und das zweite Element (120, 220) nicht geöffnet werden, und dazu ausgebildet sind, sich voneinander zu lösen, um einen Zustand herbeizuführen, in dem das erste Element (110, 210) und das zweite Element (120, 220) geöffnet und geschlossen werden können.

2. Katheterhalter (100, 200, 300) nach Anspruch 1, mit Eingriffsabschnitten (217, 227), die an dem ersten Element (110, 210) und dem zweiten Element (120, 220) vorgesehen sind, die dazu ausgebildet sind, durch einen Mechanismus, in dem das erste Element (110, 210) und das zweite Element (120, 220) geschlossen sind, ineinanderzugreifen, und die dazu ausgebildet sind, sich durch einen Mechanismus, in dem das erste Element (110, 210) und das zweite Element (120, 220) geöffnet sind, voneinander zu lösen.

3. Katheterhalter (100, 200, 300) nach Anspruch 1 oder Anspruch 2, wobei die Vielzahl von Halteabschnitten (101, 102, 103) eine Vielzahl von konkaven Abschnitten (111, 112, 113, 121, 122, 123, 311, 312, 313) aufweisen, die mit dem ungehäusten Abschnitt (10) ausgestattet werden können, bei dem die Querschnittsform desselben, die in axialer Richtung des Katheters kreuzt, eine hinterschnittene Form bildet.

## Revendications

1. Porte-cathéter (100, 200, 300) comprenant :
une partie de fixation (115) conçue pour être fixée à une partie d'un tube de support (20) destiné à loger un cathéter ; et
comprenant en outre ;
une pluralité de parties de support (101, 102, 103) formées de façon intégrante avec la partie de fixation (115) et conçues pour supporter une partie non logée (10) du cathéter, qui est découverte par rapport au tube de support (20),
où la pluralité de parties de support (101, 102, 103) comprend une pluralité de parties de prise en sandwich conçues pour prendre en sandwich la partie non logée (10) lors de la fermeture d'un premier élément (110, 210) et d'un second élément (120, 220) qui peuvent tourner librement sous une forme articulée,
**caractérisé en ce que**
ledit porte-cathéter (100, 200, 300) comprend des parties de butée (114, 124) prévues au niveau du premier élément (110, 210) et du second élément (120, 220), où les parties de butée (114, 124) sont adaptées pour venir en contact l'une avec l'autre en vue d'une restriction de manière à ne pas ouvrir le premier élément (110, 210) et le second élément (120, 220), et elles sont adaptées pour s'écarter l'une de l'autre afin d'établir un état dans lequel le premier élément (110, 210) et le second élément (120, 220) peuvent s'ouvrir et se fermer.

2. Porte-cathéter (100, 200, 300) selon la revendication 1, comprenant des parties d'engagement (217, 227) prévues au niveau du premier élément (110, 210) et du second élément (120, 220), qui sont adaptées pour venir en contact l'une avec l'autre par un mécanisme dans lequel le premier élément (110, 210) et le second élément (120, 220) sont fermés, et qui sont adaptées pour s'écarter l'une de l'autre par un mécanisme dans lequel le premier élément (110, 210) et le second élément (120, 220) sont ouverts.

3. Porte-cathéter (100, 200, 300) selon la revendication 1 ou la revendication 2, dans lequel la pluralité de parties de support (101, 102, 103) comprend une pluralité de parties concaves (111, 112, 113, 121, 122, 123, 311, 312, 313) conçues pour être adaptées sur la partie non logée (10) où la forme de section transversale de celles-ci se croisant dans la direction axiale de cathéter forme une forme évidée.
